# DEMANDE DE BREVET EUROPEEN

(11) **EP 1 837 011 A1**
(43) Date de publication de la demande: **26.09.2007**
(21) Numéro de dépôt: 07103062.1
(22) Date de dépôt: 26.02.2007
(51) Int. Cl.: A61K 8/34, A61K 8/37, A61K 8/39, A61K 8/86, A61K 8/02, A61Q 5/00, A61Q 19/00

(54) **Article cosmétique à usage unique, comprenant un support imprégné d'une lotion contenant du mono-laurate de sorbitan oxyéthylène à 4 OE.**

(30) Priorité: 28.02.2006 FR 0601757
(71) Demandeur: L'Oréal, 75008 Paris (FR)
(72) Inventeur: Masse, Virginie, 92110, CLICHY (FR)
(74) Mandataire: Bulle, Françoise

(57) **Abrégé**

La présente invention concerne un article cosmétique à usage unique comprenant au moins un support insoluble dans l'eau, imprégné d'une lotion. La lotion d'imprégnation comprend de l'eau, au moins 1% en poids d'au moins un alcool et/ou au moins un polyol cosmétiquement acceptable, et au moins 0,05 % en poids de mono-laurate de sorbitan oxyéthyléné à 4 OE.

Un autre objet de l'invention est un procédé de traitement cosmétique des matières kératiniques telles que les cheveux et/ou le cuir chevelu, mettant en oeuvre un tel article cosmétique.

## Description

La présente invention concerne un article cosmétique à usage unique, essentiellement destiné au soin des matières kératiniques, telles que notamment les cheveux ou le cuir chevelu. Plus précisément, la présente invention concerne un article cosmétique à usage unique comprenant un support insoluble dans l'eau, imprégné d'une lotion qui comprend, dans un milieu hydroalcoolique, un ester d'acide gras et de sorbitan oxyalkyléné particulier.

La présente invention concerne également un procédé de traitement cosmétique des matières kératiniques mettant en oeuvre un tel article cosmétique.

Les compositions destinées au lavage des matières kératiniques se trouvent généralement sous forme de liquides plus ou moins épais.

Ainsi la demande de brevet EP 0 115 888 décrit une composition non irritante, destinée au nettoyage de la peau, du cuir chevelu et des cheveux, et qui permet de diminuer la sécrétion de sébum liée aux lavages fréquents. Cette composition comprend de 5 à 10 % en poids d'agent émulsifiant non ionique ayant une HLB comprise entre 10 et 19, et de 0,5 à 5 % en poids d'un agent épaississant constitué de substances organiques capables de former des gels et des solutions colloïdales en présence d'eau.

La demande de brevet EP 0 461 593 décrit des compositions de nettoyage des cheveux dont les propriétés détergentes et cosmétiques sont améliorées. Cette composition comprend :
(a) de 0,05 à 15 % en poids d'un tensioactif,
(b) de 0,5 à 40 % en poids d'un alcool,
(c) de 0,1 à 25 % en poids d'eau, et
(d) de 20 à 98 % en poids d'huile liquide,

le rapport entre la viscosité du mélange comprenant les composants (a), (b) et (c) et la viscosité de l'huile (d) étant supérieur ou égal à 1.

La demande de brevet WO 03/096998 décrit des compositions de shampooings prévenant la formation des pellicules. Ces compositions contiennent comme agent actif des extraits de *Camelia sinensis,* ou leurs principes actifs du type des polyphénols (catéchines, flavonoïdes). Ces compositions peuvent également comprendre des agents antipelliculaires additionnels, et des agents tensioactifs anioniques, non ioniques amphotères, zwitterioniques et/ou cationiques. Parmi les agents tensio-actifs non ioniques, sont cités les esters d'acides gras et de sorbitan, et leurs dérivés polyoxyéthylénés comprenant de 1 à 30, et de préférence de 5 à 10 unités oxyéthylène.

Les compositions classiques sont susceptibles, lors de leur emploi, de couler dans la sphère oculaire, et de provoquer des réactions d'inconfort telles que des picotements, voire des irritations des yeux.

Par ailleurs, il est connu d'utiliser des produits cosmétiques sous forme d'un substrat imbibé d'une composition cosmétique adaptée au but recherché, en général le démaquillage et/ou le nettoyage de la peau et des yeux. Ces articles, couramment dénommés « lingettes », sont appréciés pour leur côté pratique car ils sont jetables et imprégnés de la quantité nécessaire et suffisante de produit cosmétique. L'utilisation de ces lingettes évite également la manipulation et le transport de flacons contenant des lotions ou des laits.

Ainsi, la demande de brevet WO 01/01949 décrit une composition de nettoyage personnel, telle que par exemple une composition démaquillante, qui possède des propriétés détergentes améliorées, tout en étant non irritante en particulier pour les yeux et les peaux sensibles. Cette composition comprend de 10 à 35 % en poids de silicone liquide, de 10 à 35 % en poids d'un composé dispersible dans l'eau et de 55 à 65 % en poids d'un ester. Cette composition peut être appliquée telle quelle sur la peau, ou via un support imprégné, par exemple une lingette.

La demande de brevet US 2003/0119395 décrit un article textile jetable tel qu'une lingette, qui peut être chauffé au moins deux fois sans dégradation chimique. Cet article, destiné au nettoyage de la peau, comprend une couche de base imprégnée d'une solution aqueuse laquelle comprend un parfum, au moins un tensioactif doux tel qu'un tensioactif amphotère ou un tensioactif anionique doux, au moins un agent humidifiant et au moins un agent conservateur.

La demande de brevet EP 1 405 634 décrit un article du type des lingettes sèches, destiné au nettoyage et/ou au démaquillage de la peau et/ou des yeux. Cet article comporte un substrat imprégné d'une composition substantiellement anhydre comprenant au moins 10 % en poids d'une ou plusieurs huiles, au moins un tensioactif émulsionnant et au moins un agent gélifiant hydrophile.

Enfin, la demande de brevet FR 2 867 066 décrit une composition cosmétique capillaire qui permet le nettoyage à sec des cheveux. Cette composition comprend :
(a) de 10 à 90 % en poids d'au moins un alcool en C1-C4,
(b) de 0,01 à 5 % en poids d'au moins un agent tensioactif choisi parmi les tensioactifs anioniques, non ioniques, amphotères ou zwittérioniques,
(c) de 0,01 à 5 % en poids d'un agent solubilisant les composés liposolubles, et
(d) de l'eau.

Cette composition peut être incorporée dans un article cosmétique à usage unique constitué d'un substrat imprégné d'une telle composition.

Aucun des articles jetables mentionnés ci-dessus ne permet de rendre moins agressif le nettoyage des matières kératiniques. Par ailleurs, aucun de ces articles ne permet de rendre le cuir chevelu moins sensible aux agressions extérieures.

La Demanderesse a maintenant découvert que, de manière surprenante, l'incorporation, dans un article cosmétique à usage unique tel que par exemple une lingette, d'une lotion comprenant de l'eau, au moins 1% en poids d'un alcool et/ou un polyol cosmétiquement acceptable, et au moins 0,05 % en poids d'un ester d'acide gras et de sorbitan oxyalkyléné particulier, permettait de faciliter, d'améliorer et de rendre moins agressif le nettoyage des matières kératiniques telles que notamment les cheveux et le cuir chevelu.

En particulier, une telle lingette imprégnée permet d'éviter que le produit de nettoyage ne coule dans la sphère oculaire.

De plus, de par sa composition particulière, la lotion d'imprégnation est particulièrement douce et apaisante pour le cuir chevelu. Les lingettes selon l'invention permettent ainsi, par rapport aux articles de nettoyage capillaire de l'art antérieur, d'atténuer ou de prévenir les réactions d'inconfort du cuir chevelu telles que les rougeurs, les irritations, les démangeaisons, liées aux agressions extérieures et à l'emploi répété de compositions capillaires plus ou moins agressives.

La présente invention a donc pour objet un article cosmétique à usage unique comprenant au moins un support insoluble dans l'eau, imprégné d'une lotion, laquelle comprend : de l'eau, au moins 1% en poids d'au moins un alcool et/ou au moins un polyol cosmétiquement acceptable, et au moins 0,05 % en poids de mono-laurate de sorbitan oxyéthyléné à 4 OE.

Un autre objet de l'invention est un procédé de traitement cosmétique des matières kératiniques telles que les cheveux et le cuir chevelu, mettant en oeuvre un tel article cosmétique.

D'autres objets et caractéristiques, aspects et avantages de l'invention apparaîtront plus clairement à la lecture de la description et des exemples qui suivent.

Dans le présent exposé, et de manière bien connue en soi, on désigne par « composé à X OE » un composé oxyéthyléné comprenant X unités oxyéthylène par molécule.

On désigne de plus par « composé en C_{Y} » un composé comprenant Y atomes de carbone.

Selon la présente invention, on emploie un support imprégné d'une lotion (ou « lotion d'imprégnation »), laquelle comprend de l'eau, au moins un alcool et/ou un polyol cosmétiquement acceptable, et du mono-laurate de sorbitan oxyéthyléné à 4 OE.

Par alcool et/ ou polyol cosmétiquement acceptable, on désigne un alcool et/ou un polyol compatible avec les matières kératiniques, en particulier avec le cuir chevelu, la peau, les cheveux.

Les alcools peuvent être notamment choisis parmi les alcools inférieurs en C₁-C₄, tels que par exemple l'éthanol, l'isopropanol, le tertio-butanol, le n-butanol et leurs mélanges.

Les polyols peuvent être par exemple choisis parmi le glycérol, le propylèneglycol, les polyéthylèneglycols et leurs mélanges.

On peut également employer dans le cadre de la présente invention un mélange d'alcool(s) et de polyol(s) tels que décrits ci-avant.

La lotion d'imprégnation comprend de préférence de 1 à 60 % en poids d'alcool(s) et/ou polyol(s), plus préférentiellement de 5 à 50 % en poids, mieux encore de 10 à 30 % poids, par rapport au poids total de la lotion.

Le mono-laurate de sorbitan oxyéthylène à 4 OE est par exemple commercialisé sous la dénomination TWEEN 21 par la société UNIQEMA.

La lotion d'imprégnation peut également comprendre, en plus du mono-laurate de sorbitan oxyéthyléné à 4 OE, d'autres esters d'acides gras en C₈₋₃₀ et de sorbitan oxyalkyléné, comprenant de préférence de 1 à 50 unités oxyalkylène.

Ainsi, selon un mode de réalisation avantageux, la lotion d'imprégnation comprend également du mono-laurate de sorbitan oxyéthyléné à 20 OE, tel que par exemple le produit commercialisé sous la dénomination TWEEN 20 par la société UNIQEMA.

La lotion d'imprégnation comprend de préférence de 0,05 à 10 % en poids d'ester d'acide gras et de sorbitan oxyalkyléné, plus préférentiellement de 0,1 à 8 % en poids, mieux encore de 0,2 à 5 % en poids, par rapport au poids total de la lotion.

Selon la présente invention, la lotion d'imprégnation peut également comprendre un ou plusieurs autres tensioactifs choisis parmi les tensioactifs anioniques, les tensioactifs cationiques, les tensioactifs amphotères ou zwittérioniques, et les tensioactifs non ioniques différents des esters d'acide gras et de sorbitan oxyalkylénés.

Les tensioactifs anioniques pouvant être utilisés dans lesdites lotions sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans la lotion d'imprégnation est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆-₂₄)éther-carboxyliques polyoxyalkylénés, les acides (alkyl en C₆-₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Lorsqu'ils sont présents, la quantité du ou des tensioactifs anioniques est de préférence comprise dans l'intervalle allant de 0,01 à 50 % en poids, mieux encore de 0,1 à 20% en poids par rapport au poids total de la lotion d'imprégnation.

Des exemples de tensioactifs non-ioniques utilisables dans la lotion d'imprégnation selon la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁-20)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras, les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques est de préférence comprise dans l'intervalle allant de 0,01 à 20 % en poids, plus préférentiellement de 0,1 à 10 % en poids, mieux encore de 0,1 à 5 % en poids, par rapport au poids total de la lotion d'imprégnation.

Les agents tensioactifs amphotères ou zwittérioniques, susceptibles d'être incorporés dans la lotion d'imprégnation peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (II) et (III) :

Rₐ-CONHCH₂CH₂-N(R_{b})(R_{c})(CH₂COO⁻) (II)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (III)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques est de préférence comprise dans l'intervalle allant de 0,01 à 20 % en poids, plus préférentiellement de 0,05 à 10% en poids, mieux encore de 0,1 à 2% en poids, par rapport au poids total de la lotion d'imprégnation.

A titre d'exemples de tensioactifs cationiques susceptibles d'entrer dans la composition de la lotion d'imprégnation, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline.

Lorsqu'ils sont présents, la quantité du ou des tensioactifs cationiques est de préférence comprise dans l'intervalle allant de 0,01 à 10 % en poids, plus préférentiellement de 0,2 à 5 % en poids, et encore plus préférentiellement de 0,3 à 3 % en poids, par rapport au poids total de la lotion d'imprégnation.

La quantité totale de tensioactifs est de préférence comprise dans l'intervalle allant de 0,01 à 50 % en poids, plus préférentiellement de 0,05 à 20 % en poids, mieux encore de 0,1 à 10 % en poids, par rapport au poids total de la lotion d'imprégnation.

De manière avantageuse, la lotion d'imprégnation comprend au moins un tensioactif anionique et/ou au moins un tensioactif amphotère ou zwittérionique.

Selon la présente invention, la lotion d'imprégnation peut également contenir au moins un corps gras. Les corps gras utilisables dans la présente invention sont notamment choisis parmi les huiles végétales, les huiles animales, les huiles minérales, les huiles naturelles ou synthétiques, les alcools gras, les silicones volatiles ou non, linéaires ou ramifiées, organomodifiées ou non, et les mélanges de tels corps gras.

Comme huile végétale, on peut notamment mentionner l'huile d'amande douce, l'huile d'avocat, l'huile de ricin, l'huile d'olive, la cire liquide de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de palme, l'huile de noyau d'abricot, l'huile de calophyllum, l'huile d'onagre, le beurre de karité, l'huile de son de riz, l'huile de germes de maïs, l'huile de passiflore et l'huile de seigle.

Comme huile animale, on peut notamment citer le perhydrosqualène.

Comme huile minérale, on peut notamment citer les huiles de paraffine et l'huile de vaseline.

Comme huile synthétique, on peut notamment citer le squalane, les poly(α-oléfines) comme l'isododécane ou l'isohexadécane, les huiles végétales transestérifiées et les huiles fluorées, les esters gras.

Par esters gras, on désigne les composés de formule RₐCOOR_{b} dans laquelle Rₐ représente le reste d'un acide supérieur linéaire ou ramifié, hydroxylé ou non, saturé ou non, comportant de 4 à 29 atomes de carbone et R_{b} représente une chaîne hydrocarbonée linéaire ou ramifiée, saturée ou non contenant de 3 à 30 atomes de carbone, le nombre total d'atomes de carbone de l'ester étant supérieur à 10. A titre d'exemples, on peut notamment citer l'huile de Purcellin (octanoate de stéaryle), le myristate d'isopropyle, le palmitate d'isopropyle, le stéarate de butyle, le laurate d'hexyle, l'isononanoate d'isononyle, le palmitate de 2-éthylhexyle, le laurate de 2-hexyldécyle, le palmitate de 2-octyldécyle, le myristate de 2-octyldodécyle, le néopentanoate d'isostéaryle ou le néopentanoate de tridécyle.

Les alcools gras préférés comprennent les alcools myristylique, cétylique, stéarylique, arachidylique, béhénylique et érucylique.

Les silicones préférées sont les polydiméthylsiloxanes et les polydiméthylsiloxanes aminés, sous forme d'huiles, de gommes ou de résines.

Les corps gras tels que décrits ci-dessus peuvent être présents en une quantité allant de 0,01 à 20 % en poids, de préférence de 0,1 à 10 % en poids, et mieux encore de 0,5 à 5% en poids, par rapport au poids total de la lotion d'imprégnation.

Selon l'invention, la lotion d'imprégnation peut également comprendre un ou plusieurs agent(s) antipelliculaire(s). Comme agents antipelliculaires, peuvent être employés par exemple des composés tels que la piroctone olamine, la pyrithione de zinc, l'acide salicylique, le disulfure de sélénium, et leurs mélanges.

La lotion d'imprégnation comprend alors de 0,001 à 10 % en poids d'agent(s) antipelliculaire(s), de préférence de 0,1 à 5 % en poids, encore plus préférentiellement de 0,2 à 2 % en poids, par rapport au poids total de ladite lotion.

La lotion d'imprégnation peut comprendre en outre un ou plusieurs additifs classiques bien connus dans la technique, tels que les agents apaisants, les agents antichute, les agents anti-âge, les agents oxydants, les vitamines et pro-vitamines dont le panthénol, les céramides et pseudo-céramides, les filtres solaires, les pigments minéraux ou organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

L'homme de métier veillera à choisir les éventuels additifs et leurs quantités de manière à ce qu'ils ne nuisent pas aux propriétés des articles cosmétiques objets de la présente invention.

Ces additifs peuvent être présents dans la lotion d'imprégnation en une quantité allant de 0 à 20 % en poids par rapport au poids total de ladite lotion.

Le support entrant dans la composition des articles cosmétiques selon l'invention doit être insoluble dans l'eau.

Ce support peut comprendre une ou plusieurs couches qui peuvent être choisies dans le groupe comprenant les matériaux tissés, les matériaux non-tissés, les mousses, les éponges, les ouates, ces matériaux pouvant se présenter par exemple sous forme de feuilles, de boules, de films.

De préférence, le support comprend au moins une couche constituée d'un matériau en non tissé. Un tel matériau peut être à base de fibres d'origine naturelle (lin, laine, coton, soie) ou d'origine synthétique (dérivés de cellulose, viscose, dérivés polyvinyliques, polyesters comme le théréphtalate de polyéthylène, polyoléfines comme le polyéthylène ou le polypropylène, polyamides comme le Nylon, dérivés acryliques). Les non tissés sont décrits de façon générale dans RIEDEL « Nonwoven Bonding Methods & Materials », Nonwoven World (1987). Ces matériaux sont obtenus selon les procédés usuels de la technique de préparation des non-tissés.

Quand le support est un non-tissé, on utilise de préférence un non-tissé suffisamment épais, qui ne se met pas en boule et qui est assez solide pour ne pas se déliter et ne pas pelucher à l'application sur le cuir chevelu. Il doit être absorbant, et de préférence doux au moins sur une face.

Comme non-tissés appropriés, on peut citer par exemple ceux commercialisés sous les dénominations Ultraloft 15285-01, Ultraloft 182-008, Ultraloft 182-010, Ultraloft 182-016 par la Société BBA, Vilmed M1519 Blau, Vilmed M 1550 et 112-132-3 par la Société Freudenberg, celui commercialisé sous la dénomination Norafin 11601-010B par la Société Jacob Holm Industries, les non tissés flockés commercialisés sous les dénominations Univel 109 et Univel 119 par la Société Uni Flockage, les non tissés commercialisés sous la dénomination Sawatex par la société Sandler.

Le support peut comporter une ou plusieurs couches ayant des propriétés identiques ou différentes et avoir des propriétés d'élasticité, de douceur et autres appropriées à l'usage recherché. Les supports peuvent comporter par exemple deux parties ayant des propriétés d'élasticité différentes comme décrit dans le document WO-A-99/13861, ou comporter une seule couche avec des densités différentes comme décrit dans le document WO-A-99/25318, ou comporter deux couches de textures différentes comme décrit dans le document WO-A-98/18441.

De préférence, le support comprend au moins une couche de grammage compris dans l'intervalle allant de 30 à 90 g/m², plus préférentiellement de 40 à 70 g/m².

Le support peut avoir toute taille et toute forme appropriées au but recherché. Il peut ainsi avoir par exemple la forme d'une lingette rectangulaire, ou la forme d'un gant ou d'une moufle, faciles à enfiler sur la main, ou la forme d'une compresse ronde. Il a généralement une surface comprise entre 0,005 m² et 0,1 m², de préférence entre 0,01 m² et 0,05 m².

Le taux d'imprégnation en poids du support par la lotion est avantageusement compris entre 10 et 1500 %, de préférence entre 50 et 500 %, mieux encore entre 100 et 400 %.

Les techniques d'imprégnation d'un support par une lotion sont bien connues dans ce domaine, et sont toutes applicables à la présente invention. En général, la lotion d'imprégnation est ajoutée au support au moyen d'une ou plusieurs techniques comprenant l'immersion, l'enduction, la vaporisation, etc.

L'invention a également pour objet un procédé de traitement cosmétique des matières kératiniques, par exemple un procédé de nettoyage ou de soin cosmétique des matières kératiniques, consistant à passer sur lesdites matières un article cosmétique tel que décrit ci-dessus, puis à éventuellement rincer après un éventuel temps de pose.

Selon un mode de réalisation, les articles cosmétiques selon l'invention sont utilisés en non rincé, c'est-à-dire que le procédé ci-avant ne comprend pas d'étape de rinçage. Ce mode de réalisation est particulièrement avantageux dans la mesure où il permet d'éviter de manière sûre tout contact de la lotion d'imprégnation avec les yeux.

De préférence, les matières kératiniques sont les cheveux et/ou le cuir chevelu.

La présente invention concerne également l'utilisation d'un article cosmétique tel que décrit ci-avant, pour le nettoyage ou le soin des matières kératiniques, en particulier des cheveux et/ou du cuir chevelu.

La présente invention concerne enfin l'utilisation cosmétique d'un article cosmétique tel que décrit ci-avant, afin d'atténuer les réactions d'inconfort du cuir chevelu

L'exemple suivant est donné à titre illustratif de la présente invention, et ne saurait en limiter la portée.

### EXEMPLE

Ont été préparés des articles cosmétiques conformes à l'invention, comprenant les lotions A et B de composition détaillée dans le tableau ci-dessous.

Dans le tableau ci-après, toutes les quantités sont indiquées en pour cent en poids de matière active par rapport au poids total de la lotion.

| Composition de la lotion | A | B |
|---|---|---|
| alcool éthylique à 96 degrés | 15,8 | 15,8 |
| mélange d'hydrocarbures isoparaffiniques (1) | 1 | 1 |
| mono-laurate de sorbitan oxyéthyléné (4 OE) (2) | 0,25 | 0,5 |
| N-cocoyl amidoéthyl, N-éthoxycarboxyméthyl glycinate de sodium (3) | 0,2 | - |
| p-hydroxybenzoate de méthyle | 0,2 | 0,2 |
| parfum | 0,1 | 0,1 |
| eau qsp | 100% | 100% |

| | | |
|---|---|---|
| (1) commercialisé sous la dénomination ISOPAR H par la société EXXON MOBIL. (2) commercialisé sous la dénomination TWEEN 21 par la société UNIQEMA. (3) commercialisé sous la dénomination MIRANOL C2M par la société RHODIA CHIMIE. | | |

Des lingettes ont été préparées à partir de chacune des ces lotions, en imprégnant un support en non tissé commercialisé sous la dénomination SAWATEX 2647 par la société SANDLER, avec un taux d'imprégnation en poids du support par la lotion de 250 %. Ce support présente un grammage d'environ 55 g/m², est constitué de fibres de viscose et de polypropylène, et a été utilisé sous forme de gants.

Les performances des lingettes décrites ci-avant ont été évaluées par des professionnels, sur des panels de modèles, en appliquant la lingette sur les cheveux secs et le cuir chevelu, en massant légèrement.

Après application, les cheveux sèchent très rapidement. Ils sont propres et doux, et le cuir chevelu n'est pas irrité.

## Revendications

1. Article cosmétique à usage unique comprenant au moins un support insoluble dans l'eau, imprégné d'une lotion, laquelle comprend : de l'eau, au moins 1% en poids d'au moins un alcool et/ou au moins un polyol cosmétiquement acceptable, et au moins 0,05 % en poids de mono-laurate de sorbitan oxyéthyléné à 4 OE, par rapport au poids total de la lotion.

2. Article cosmétique selon la revendication 1, **caractérisé en ce que** la lotion d'imprégnation comprend au moins un alcool choisi parmi les alcools inférieurs en C₁-C₄.

3. Article cosmétique selon l'une quelconque des revendications précédentes, **caractérisé en ce que** la lotion d'imprégnation comprend au moins un polyol choisi parmi le glycérol, le propylèneglycol, les polyéthylèneglycols et leurs mélanges.

4. Article cosmétique selon l'une quelconque des revendications précédentes, **caractérisé en ce que** la lotion d'imprégnation comprend de 1 à 60 % en poids d'alcool(s) et/ou de polyol(s), par rapport au poids total de la lotion.

5. Article cosmétique selon la revendication précédente, **caractérisé en ce que** la lotion d'imprégnation comprend de 5 à 50 % en poids d'alcool(s) et/ou de polyol(s), de préférence de 10 à 30 % poids, par rapport au poids total de la lotion.

6. Article cosmétique selon l'une quelconque des revendications précédentes, **caractérisé en ce que** la lotion d'imprégnation comprend également du mono-laurate de sorbitan oxyéthyléné à 20 OE.

7. Article cosmétique selon l'une quelconque des revendications précédentes, **caractérisé en ce que** la lotion d'imprégnation comprend de 0,05 à 10 % en poids d'ester d'acide gras et de sorbitan oxyalkyléné, par rapport au poids total de la lotion.

8. Article cosmétique selon la revendication précédente, **caractérisé en ce que** la lotion d'imprégnation comprend de 0,1 à 8 % en poids d'ester d'acide gras et de sorbitan oxyalkyléné, de préférence de 0,2 à 5 % en poids, par rapport au poids total de la lotion.

9. Article cosmétique selon l'une quelconque des revendications précédentes, **caractérisé en ce que** la lotion d'imprégnation comprend un ou plusieurs autres tensioactifs choisis parmi les tensioactifs anioniques, les tensioactifs cationiques, les tensioactifs amphotères ou zwittérioniques, et les tensioactifs non ioniques différents des esters d'acide gras et de sorbitan oxyalkylénés.

10. Article cosmétique selon la revendication précédente, **caractérisé en ce que** la lotion d'imprégnation comprend au moins un tensioactif anionique choisi parmi les sels, en particulier les sels de métaux alcalins, les sels d'ammonium, les sels d'amines, les sels d'aminoalcools ou les sels de métaux alcalino-terreux, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

11. Article cosmétique selon la revendication 9, **caractérisé en ce que** la lotion d'imprégnation comprend au moins un tensioactif anionique choisi parmi les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

12. Article cosmétique selon la revendication 9, **caractérisé en ce que** la lotion d'imprégnation comprend au moins un tensioactif anionique choisi parmi les acyl-lactylates dont le groupe acyle comporte de 8 à 20 atomes de carbone, les acides alkyl-D-galactoside-uroniques et leurs sels, ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène.

13. Article cosmétique selon l'une quelconque des revendications 9 à 12, **caractérisé en ce que** la quantité du ou des tensioactifs anioniques est comprise dans l'intervalle allant de 0,01 à 50 % en poids, de préférence de 0,1 à 20% en poids, par rapport au poids total de la lotion d'imprégnation.

14. Article cosmétique selon la revendication 9, **caractérisé en ce que** la lotion d'imprégnation comprend au moins un tensioactif amphotère ou zwittérionique choisi parmi les dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate.

15. Article cosmétique selon la revendication 9, **caractérisé en ce que** la lotion d'imprégnation comprend au moins un tensioactif amphotère ou zwittérionique choisi parmi les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïncs.

16. Article cosmétique selon l'une quelconque des revendications 14 et/ou 15, **caractérisé en ce que** la quantité du ou des tensioactifs amphotères ou zwittérioniques est comprise dans l'intervalle allant de 0,01 à 20 % en poids, plus préférentiellement de 0,05 à 10 % en poids, mieux encore de 0,1 à 2 % en poids, par rapport au poids total de la lotion d'imprégnation.

17. Article cosmétique selon la revendication 9, **caractérisé en ce que** la lotion d'imprégnation comprend au moins un tensioactif cationique, choisi parmi les sels d'amines grasses primaires, secondaires ou tertiaires, éventuellement polyoxyalkylénées; les sels d'ammonium quaternaire; les dérivés d'imidazoline.

18. Article cosmétique selon la revendication précédente, **caractérisé en ce que** la quantité du ou des tensioactifs cationiques est comprise dans l'intervalle allant de 0,01 à 10 % en poids, plus préférentiellement de 0,2 à 5 % en poids, par rapport au poids total de la lotion d'imprégnation.

19. Article cosmétique selon la revendication 9, **caractérisé en ce que** la lotion d'imprégnation comprend au moins un tensioactif non ionique, choisi parmi les alcools, les alpha-diols, les alkyl(C₁₋₂₀)phénols ou les acides gras polyéthoxylés, polypropoxylés ou polyglycérolés, les condensats d'oxyde d'éthylène et d'oxyde de propylène sur des alcools gras, les amides gras polyéthoxylés, les amides gras polyglycérolés comportant en moyenne de 1 à 5 groupements glycérol, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

20. Article cosmétique selon la revendication précédente, **caractérisé en ce que** la quantité du ou des tensioactifs non ioniques est comprise dans l'intervalle allant de 0,01 à 20 % en poids, plus préférentiellement de 0,1 à 10 % en poids, mieux encore de 0,1 à 5% en poids, par rapport au poids total de la lotion d'imprégnation.

21. Article cosmétique selon l'une quelconque des revendications 9 à 20, **caractérisé en ce que** quantité totale de tensioactifs est comprise dans l'intervalle allant de 0,01 à 50 % en poids, plus préférentiellement de 0,05 à 20 % en poids, mieux encore de 0,1 à 10 % en poids, par rapport au poids total de la lotion d'imprégnation.

22. Article cosmétique selon l'une quelconque des revendications précédentes, **caractérisé en ce que** la lotion d'imprégnation contient également au moins un corps gras, choisi parmi les huiles végétales, les huiles animales, les huiles minérales, les huiles naturelles ou synthétiques, les alcools gras, les silicones volatiles ou non, linéaires ou ramifiées, organomodifiées ou non, et les mélanges de tels corps gras.

23. Article cosmétique selon la revendication précédente, **caractérisé en ce que** la lotion d'imprégnation comprend de 0,01 à 20 % en poids de corps gras, de préférence de 0,1 à 10 % en poids, par rapport au poids total de la lotion d'imprégnation.

24. Article cosmétique selon l'une quelconque des revendications précédentes, **caractérisé en ce que** la lotion d'imprégnation comprend en outre de 0,001 à 10 % en poids d'au moins un agent antipelliculaire.

25. Article cosmétique selon l'une quelconque des revendications précédentes, **caractérisé en ce que** la lotion d'imprégnation comprend en outre un ou plusieurs additifs classiques, choisis parmi les agents apaisants, les agents antichute, les agents anti-âge, les agents oxydants, les vitamines et pro-vitamines, les céramides et pseudo-céramides, les filtres solaires, les pigments minéraux ou organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

26. Article cosmétique selon l'une quelconque des revendications précédentes, **caractérisé en ce que** le support comprend une ou plusieurs couches choisies dans le groupe comprenant les matériaux tissés, les matériaux non-tissés, les mousses, les éponges, les ouates.

27. Article cosmétique selon la revendication précédente, **caractérisé en ce que** le support comprend au moins une couche constituée d'un matériau en non tissé, à base de fibres d'origine naturelle ou d'origine synthétique.

28. Article cosmétique selon l'une quelconque des revendications précédentes, **caractérisé en ce que** le support comprend au moins une couche de grammage compris dans l'intervalle allant de 30 à 90 g/m², de préférence de 40 à 70 g/m².

29. Article cosmétique selon l'une quelconque des revendications précédentes, **caractérisé en ce que** le support présente la forme d'une lingette rectangulaire, la forme d'un gant ou d'une moufle, ou la forme d'une compresse ronde.

30. Article cosmétique selon l'une quelconque des revendications précédentes, **caractérisé en ce que** le support possède une surface comprise entre 0,005 m² et 0,1 m², de préférence entre 0,01 m² et 0,05 m².

31. Article cosmétique selon l'une quelconque des revendications précédentes, **caractérisé en ce que** le taux d'imprégnation en poids du support par la lotion est compris entre 10 et 1500 %.

32. Article cosmétique selon la revendication précédente, **caractérisé en ce que** le taux d'imprégnation en poids du support par la lotion est compris entre 50 et 500 %, de préférence entre 100 et 400 %.

33. Procédé de traitement cosmétique des matières kératiniques, consistant à passer sur lesdites matières un article cosmétique selon l'une quelconque des revendications 1 à 32, puis à éventuellement rincer après un éventuel temps de pose.

34. Procédé selon la revendication précédente, **caractérisé en ce qu'**il ne comprend pas d'étape de rinçage.

35. Utilisation d'un article cosmétique selon l'une quelconque des revendications 1 à 32, pour le nettoyage ou le soin des matières kératiniques.

36. Utilisation d'un article cosmétique selon l'une quelconque des revendications 1 à 32, pour le nettoyage ou le soin des cheveux et/ou du cuir chevelu.

37. Utilisation cosmétique d'un article selon l'une quelconque des revendications 1 à 32, afin d'atténuer les réactions d'inconfort du cuir chevelu.
